# EUROPEAN PATENT SPECIFICATION

(11) **EP 1 389 955 B1**
(45) Date of publication and mention of the grant of the patent: **28.10.2009**
(21) Application number: 02742821.8
(22) Date of filing: 06.05.2002
(51) Int. Cl.: A61B 5/15, A61B 10/00, B23K 26/38

(54) **DEVICE FOR SAMPLING SMALL AND PRECISE VOLUMES OF LIQUID**
VORRICHTUNG ZUR PROBEENTNAHME KLEINER UND GENAUER FLÜSSIGKEITSVOLUMEN
DISPOSITIF POUR LE PRELEVEMENT D'UN ECHANTILLON DE VOLUME DE LIQUIDE FAIBLE ET PRECIS

(30) Priority: 10.05.2001 DK 200100731
(43) Date of publication of application: 25.02.2004
(73) Proprietor: Chempaq A/S, 3520 Farum (DK)
(72) Inventor: LARSEN, Ulrik, Darling, DK-2800 Kgs. Lyngby (DK)
(74) Representative: Jakobsen, Gert Hoey
(86) International application number: PCT/DK2002/000292
(87) International publication number: WO 2002/089670

(56) References cited:
- EP-A- 0 005 979
- EP-A- 1 240 944
- WO-A-01/13795
- DE-A- 3 507 032
- US-A- 3 848 581
- US-A- 5 833 630
- US-B1- 6 218 193

## Description

The present invention relates to precise sampling of small volumes of liquid, for example body liquids, such as blood, semen, saliva, spinal fluid, lymph, perspiration, urine, etc.

In order to determine the composition of a liquid, a sample of the liquid is typically subjected to various measurements, e.g. in order to determine the concentration of constituents of the liquid with a certain precision requiring that the withdrawn volume of the sample must be repeatable. With present sampling methods, the precision of the sample volume deteriorates as the volume become smaller; the smaller volume the poorer precision, thus leading to a low precision in concentration determination of constituents in the sample. For sample volumes less than 1 µL, this problem is significant; even high precision pipettes have a reasonably unrefined precision in this region. Typically, surface variations and dirt at the pipette tip cause relatively large variations in sampled volume. Another rather commonly used method for sampling of small volumes, typically in the order of 10-20 µL, is the use of capillary tubes. The liquid is drawn into the interior of the capillary tube by capillary action. Variations in the sample volume occurs due to variations at either end of the tube; at the sample-taking end of the capillary tube variations occurs due to liquid sticking; at the opposing end variations in the filling occurs due to small differences in the liquid surface tension at the end of the tube. These variations become even more significant when the required sample volume becomes smaller than 10 µL.

DE 35 07 032 relates to an apparatus for volumetric proportioning and transferring of a sample into a measuring vessel while excluding air. The apparatus has a movable member with a sampling bore enabling the entrapment of small volumes of liquid.

US 6,218,193 relates to a complex apparatus for high precision, small volume processing of fluids, comprising one or more open ended capillary tubes. The apparatus comprises a pneumatic piston and position sensors for moving and controlling the fluid in the capillary tube. The movement of the pneumatic piston is controlled by a computer in response to the output of the sensors in order to determine a precise small volume of liquid present in the capillary tube.

WO 01/13795 relates to a disposable test vial kit with sample delivery device. The kit comprises several parts to be separated and assembled during a sample test. The test kit comprises a sample delivery member, which may be an open ended capillary tube for obtaining a sample.

It is an object of the present invention to provide a device for sampling small volumes of liquid, such as volumes less than 1 to 10 µL, with a high precision.

According to the present invention, the above-mentioned and other objects are fulfilled by a device for sampling a small and precise volume of liquid, comprising a first member with a first opening for entrance of a liquid sample into a first cavity forming a capillary tunnel in the first member and with a second opening for outputting the liquid sample from the first cavity, and a second member with a second cavity forming a capillary tunnel for receiving and holding the liquid sample and having a third opening communicating with the second cavity, the second member being movably positioned in relation to the first member in such a way that, in a first position, the second opening is in communication with the third opening for entrance of the liquid sample into the second cavity, and, in a second position, the third opening is closed so that a liquid sample is entrapped in the closed second cavity, and wherein the capillary tunnel includes one or more hydrophilic surface coatings.

The first opening of the first member may be brought into contact with a liquid to be sampled so that the liquid may flow through the first opening into the first cavity and out of the second opening. The second member is movably positioned in relation to the first member. During sampling of the liquid, the second member is positioned in a first position in relation to the first member in which first position, the second opening is in communication with the third opening so that sampled liquid may flow through the second and third opening into the second cavity. The third opening may be disconnected from the second opening in a second position of the second member in relation to the first member in such a way that the third opening is closed, e.g. by the first member, in the second position of the second member.

This entrapment of the liquid sample in the closed second cavity eliminates the effect of the variations in adherence and filling of liquid at sample device openings that is believed to cause the poor precision of small sample volumes in known liquid sampling devices and thus leads to a sampling device with an improved sampling precision.

Further, the first member may have a fourth cavity with fifth and sixth openings, and the second member may have a fourth opening so that, in the first position, the fourth opening communicates with the fifth opening, and the first opening communicates with the sixth opening so that the combined first and second cavities extends through the first and the second member and communicates with the environment through the first and the sixth opening. Thus, air may escape from the combined cavity through the sixth opening. Preferably, in the first position, a part of the liquid entering the second cavity may leave the second cavity through the fourth opening thereby ensuring that the second cavity is completely filled with liquid during liquid sampling whereby the risk of sampling with a reduced sample volume leading to low accuracy sampling is significantly reduced.

The second member may be inserted into the first member. For example, the first member may comprise a third cavity for receiving and accommodating at least a part of the second member.

The second member may have a cylindrical shape. A cylindrical shape facilitates displacement of the second member along a longitudinal axis of the cylinder. For example, a cylindrical second member may be inserted into a hole with a matching cross-section in the first member for displacement between the first and second position along a longitudinal axis of the second member.

The second member may have a circular cross-section, for example the second member may have a circular cylindrical shape. A circular cross-section facilitates displacement of the second member by rotation of the member around a centre axis of the circular cross-section. For example, a circular cylindrical second member may be inserted into a matching circular hole in the first member for displacement between the first and second position along a longitudinal axis of the second member, or, by rotation around a centre axis of the circular cylinder, or, by a combination of the displacement and the rotation.

Liquid to be sampled may enter the cavities by any force causing a liquid flow, such as capillary action, diffusion, osmosis, pressure, suction, gravity, flow injection, liquid carrier, etc.

The first cavity forms a first capillary tunnel for entrance of a liquid sample by capillary attraction. The capillary tunnel is dimensioned so that, upon contact between the first opening and liquid to be sampled, a sample of the liquid is drawn into the first opening and the first capillary tunnel and the second opening by capillary attraction.

Further, the second cavity forms a second capillary tunnel adapted for drawing the liquid sample into the second cavity by capillary attraction. Preferably, the first and second capillary tunnel has the same diameter, and it is also preferred that, in the first position, the first and second capillary tunnel extend along substantially the same longitudinal centre axis.

In an embodiment of the present invention, the second member is rotatable about an axis of rotation that is substantially perpendicular to a longitudinal axis of the second cavity, and/or the second member may be displaced in a direction substantially perpendicular to a longitudinal axis of the second cavity.

The liquid sample may be brought into contact with another liquid after displacement of the second member, e.g. by emptying the second cavity through the fourth opening in the second member by any force causing a liquid flow, such as diffusion, osmosis, pressure, suction, gravity, flow injection, liquid carrier, etc.

Further, the liquid sample may be brought into contact with a selected liquid of a plurality of liquids after displacement of the second member into a corresponding selected position of a corresponding plurality of positions.

The surface of the first and second inner capillary tunnel walls is hydrophilic whereby the capillary attraction of the liquid sample is facilitated. For example, the inner tunnel walls may be made of e.g. glass or polymers, such as polystyrene.

The capillary tunnel walls are covalently or non-covalently coated with a hydrophilic material, such as a polymer or one or more reagents, and may be made of another type of material.

The capillary tunnel may also include one or more reagents adhered or chemically bonded to the inner tunnel wall. These reagents serve the purposes of further facilitating the capillary attraction of the sample and causing a chemical reaction in the liquid sample, e.g. introducing anticoagulant activity in a blood sample. Such reagents may comprise heparin, salts of EDTA, etc.

Preferably, the second member is made of a polymer.

For a better understanding of the present invention reference will now be made, by way of example, to the accompanying drawings, in which:
Fig. 1 shows schematically a preferred embodiment of the invention,
Fig. 2 shows schematically the operation of the embodiment shown in Fig. 1, and
Fig. 3 shows schematically the operation of another embodiment of the invention.

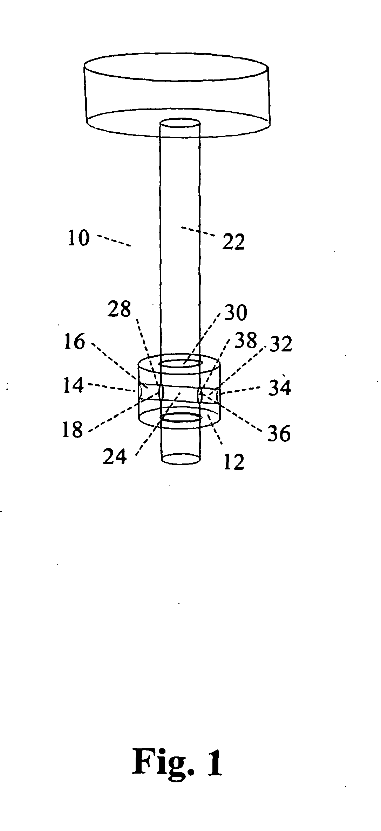

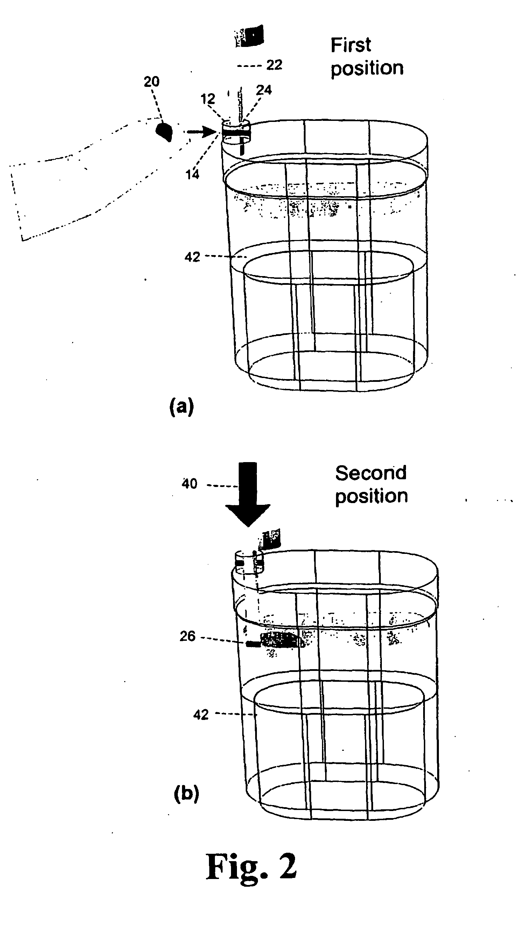

Fig. 1 schematically illustrates a device for sampling a small and accurate volume of liquid in accordance with the present invention. The device 10 comprises a first member 12 with a first opening 14 for entrance of a liquid sample (not shown) into a first cavity 16 in the first member 12 and with a second opening 18 for outputting the liquid sample from the first cavity 16. The first cavity 16 forms a capillary tunnel. The first opening 14 of the first member 12 may be brought into contact with a liquid 20 (shown in Fig. 2) to be sampled so that the liquid 20 may flow through the first opening 14 into the first cavity 16 and out of the second opening 18 by capillary attraction. The device 10 further comprises a second member 22 with a second cavity 24 for receiving and holding the liquid sample 26 (shown in Fig. 2) and having a third opening 28 communicating with the second cavity 24. The second cavity forms a capillary tunnel with the same diameter as the first cavity 16. The second member 22 is a circular cylinder that is movably positioned in relation to the first member 12. During sampling of the liquid, the second member 22 is positioned in the illustrated first position in relation to the first member 12 wherein the second opening 18 is in communication with the third opening 28 so that sampled liquid may flow through the second 18 and third opening 28 into the second cavity 24 by capillary attraction. The third opening 28 may be disconnected from the second opening 18 in a second position of the second member 22 in relation to the first member 12 so that the liquid sample 26 contained in the second cavity 24 is disconnected from the first cavity 16.

The second member 22 is inserted into a third cavity 30 of the first member 12 for receiving and accommodating a part of the second member 22. The second member 22 may be displaced between the first and second position along a longitudinal axis of the second member 22 that is also substantially perpendicular to a longitudinal axis of the second cavity 24. The second member 22 may also be rotatable about a longitudinal axis that is substantially perpendicular to a longitudinal axis of the second cavity 24. In the first position, the first 16 and second 24 capillary tunnels extend along substantially the same longitudinal centre axis.

Example 1: The capillary tunnel forming the second cavity 24 may have a length of 8 mm and a diameter of 0.9 mm for containing a liquid sample of 5.089 µL.

Example 2: The capillary tunnel forming the second cavity 24 may have a length of 5 mm and a diameter of 0.5 mm for containing a liquid sample of 0.982 µL.

Example 3: The capillary tunnel forming the second cavity 24 may have a length of 3 mm and a diameter of 0.3 mm for containing a liquid sample of 0.212 µL.

In the illustrated embodiment the first member 12 is symmetrical and has a fourth cavity 32 with openings 34, 36 opposite the first cavity 16, and the second member 22 has an opening 38 opposite the opening 28 so that, in the first position, a capillary tunnel extends through the first 12 and the second 22 member and communicates with the environment through openings 14, 36. Thus, air may escape from the capillary tunnel through opening 36. Further, in the first position, a part of the liquid entering the second cavity 24 will leave the cavity 24 through opening 38 thereby ensuring that the cavity 24 has been completely filled with liquid during liquid sampling eliminating the risk of sampling with a reduced sample volume leading to low accuracy sampling.

Fig. 2 schematically illustrates the operating principle of the embodiment of the present invention shown in Fig. 1. In Fig. 2a, the second member 22 is in its first position, and a sample of the liquid 20 is drawn into the capillary tunnel as described above with reference to Fig. 1. In Fig. 2b, the second member 24 has been displaced to its second position as indicated by the arrow 40, and in this position the liquid sample 26 may be brought into contact with another liquid, e.g. for analysing purposes. The container 42 may have a plurality of compartments containing different liquids so that the liquid sample 26 may be brought into contact with a selected liquid of a plurality of liquids after displacement of the second member 22 into a corresponding selected position of a corresponding plurality of positions.

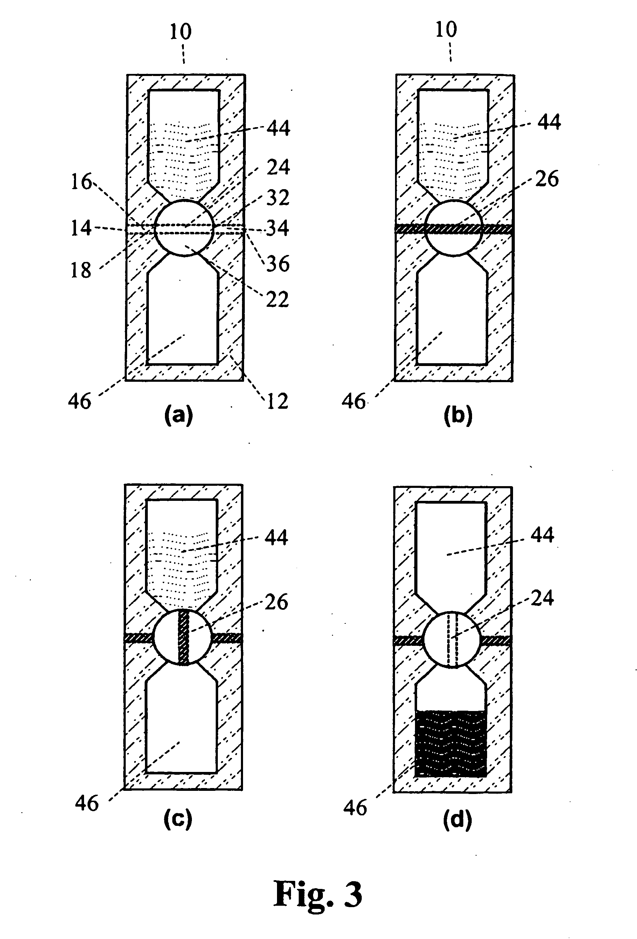

Fig. 3 schematically illustrates another embodiment of the invention and its operating principle. The illustrated device 10 also includes a chamber 44 for storing a diluent for diluting the sample and a mixing chamber 46 for mixing the sample 26 and the diluent. Fig. 3a illustrates the device 10 ready for receiving the liquid. In Fig. 3b, a sample has entered into the capillary tunnel, and in Fig. 3c the second member 22 has been rotated into the second position for isolation of an accurate volume of the sample 26, and finally Fig. 3d illustrates that the sample 26 has been washed out of the capillary tunnel 24 and into the mixing chamber 46 by the diluent.

Although the principles of the present invention have been explained above with reference to a device utilising capillary attraction, it is obvious that the invention may as well be embodied in a syringe, a pipette, etc. The invention does not depend on the forces or principles utilised to introduce the liquid sample in the device. It is the gist of the invention that a part of the sampled liquid is entrapped with a high precision.

## Claims

1. A device (10) for sampling a small and accurate volume of liquid, comprising
a first member (12) with a first opening (14) for entrance of a liquid sample into a first cavity (16) forming a capillary tunnel in the first member (12) and with a second opening (18) for outputting the liquid sample from the first cavity (16), and
a second member (22) with a second cavity (24) forming a capillary tunnel for receiving and holding the liquid sample and having a third opening (28) communicating with the second cavity (24), the second member being movably positioned in relation to the first member (12) in such a way that, in a first position, the second opening (18) is in communication with the third opening (28) for entrance of the liquid sample into the second cavity, and, in a second position, the third opening is closed so that a liquid sample is entrapped in the closed second cavity,
**characterized in that**
the capillary tunnel includes one or more hydrophilic surface coatings.

2. A device (10) according to claim 1, **characterized in that** the coating is a reagent.

3. A device (10) according to claim 2, **characterized in that** the reagent is an anticoagulant.

4. A device (10) according to claim 3, **characterized in that** the reagent comprises heparin.

5. A device (10) according to claim 3, **characterized in that** the reagent comprises salt of EDTA.

6. A device (10) according to any of the preceding claims, **characterized in that** the coating is adhered to the inner tunnel wall.

7. A device (10) according to any of the preceding claims, **characterized in that** the coating is chemically bonded to the inner tunnel wall.

8. A device (10) according to claim any of the preceding claims, **characterized in that** the first member further comprises a third cavity (30) for receiving and accommodating at least a part of the second member.

9. A device (10) according to any of the preceding claims, **characterized in that** the second member has a cylindrical shape.

10. A device (10) according to claim 8, **characterized in that** the second member has a circular cylindrical shape.

11. A device (10) according to any of the preceding claims, **characterized in that** the second member is rotatable about an axis of rotation that is substantially perpendicular to a longitudinal axis of the second cavity.

12. A device (10) according to any of the preceding claims, **characterized in that** the second member may be displaced in a direction substantially perpendicular to a longitudinal axis of the second cavity.

13. A device (10) according to any of the preceding claims, **characterized in that** the liquid sample may be brought into contact with another liquid after displacement of the second member.

14. A device (10) according to claim 13, **characterized in that** the liquid sample may be brought into contact with a selected liquid of a plurality of liquids after displacement of the second member into a corresponding selected position of a corresponding plurality of positions.

15. A device (10) according to any of the preceding claims, **characterized in that** the second member is made of a polymer.

## Patentansprüche

1. Vorrichtung (10) zur Probenentnahme eines kleinen und genauen Flüssigkeitsvolumens, mit
einem ersten Teil (12), der eine erste Öffnung (14) für den Eintritt einer Flüssigkeitsprobe in einen ersten Hohlraum (16), welcher einen Kapillartunnel in dem ersten Teil (12) bildet, und eine zweite Öffnung (18) zum Ausgeben der Flüssigkeitsprobe aus dem ersten Hohlraum (16) aufweist, und
einem zweiten Teil (22), der einen zweiten Hohlraum (24) aufweist, welcher einen Kapillartunnel zum Aufnehmen und Halten der Flüssigkeitsprobe bildet, und der eine dritte Öffnung (28) hat, die mit dem zweiten Hohlraum (24) kommuniziert, wobei das zweite Teil relativ zu dem ersten Teil (12) derart beweglich positioniert ist, dass in einer ersten Position die zweite Öffnung (18) mit der dritten Öffnung (28) kommuniziert, um die Flüssigkeitsprobe in den zweiten Hohlraum eintreten zu lassen, und in der zweiten Position die dritte Öffnung geschlossen ist, so dass eine Flüssigkeitsprobe in dem geschlossenen zweiten Hohlraum eingeschlossen ist,
**dadurch gekennzeichnet, dass**
der Kapillartunnel eine oder mehrere hydrophile Oberflächenbeschichtungen aufweist.

2. Vorrichtung (10) nach Anspruch 1, **dadurch gekennzeichnet, dass** die Beschichtung ein Reagens ist.

3. Vorrichtung (10) nach Anspruch 2, **dadurch gekennzeichnet, dass** das Reagens ein Antikoagulans ist.

4. Vorrichtung (10) nach Anspruch 3, **dadurch gekennzeichnet, dass** das Reagens Heparin aufweist.

5. Vorrichtung (10) nach Anspruch 3, **dadurch gekennzeichnet, dass** das Reagens EDTA-Salz aufweist.

6. Vorrichtung (10) nach einem der vorhergehenden Ansprüche, **dadurch gekennzeichnet, dass** die Beschichtung an der inneren Tunnelwand haftet.

7. Vorrichtung (10) nach einem der vorhergehenden Ansprüche, **dadurch gekennzeichnet, dass** die Beschichtung chemisch mit der inneren Tunnelwand verbondet ist.

8. Vorrichtung (10) nach einem der vorhergehenden Ansprüche, **dadurch gekennzeichnet, dass** das erste Teil ferner einen dritten Hohlraum (30) zum Aufnehmen und Halten mindestens eines Teilbereichs des zweiten Teils aufweist.

9. Vorrichtung (10) nach einem der vorhergehenden Ansprüche**, dadurch gekennzeichnet, dass** das zweite Teil eine zylindrische Form hat.

10. Vorrichtung (10) nach Anspruch 8, **dadurch gekennzeichnet, dass** das zweite Teil eine kreiszylindrische Form hat.

11. Vorrichtung (10) nach einem der vorhergehenden Ansprüche, **dadurch gekennzeichnet, dass** das zweite Teil um eine Drehachse drehbar ist, die im Wesentlichen rechtwinklig zu einer Längsachse des zweiten Hohlraums verläuft.

12. Vorrichtung (10) nach einem der vorhergehenden Ansprüche, **dadurch gekennzeichnet, dass** das zweite Teil in einer Richtung verlagert werden kann, die im Wesentlichen rechtwinklig zu einer Längsachse des zweiten Hohlraums verläuft.

13. Vorrichtung (10) nach einem der vorhergehenden Ansprüche, **dadurch gekennzeichnet, dass** nach der Verlagerung des zweiten Teils die Flüssigkeitsprobe in Kontakt mit einer anderen Flüssigkeit gebracht werden kann.

14. Vorrichtung (10) nach Anspruch 13, **dadurch gekennzeichnet, dass** nach der Verlagerung des zweiten Teils in eine entsprechende gewählte Position aus einer entsprechenden Mehrzahl von Positionen die Flüssigkeitsprobe in Kontakt mit einer gewählten Flüssigkeit aus einer Mehrzahl von Flüssigkeiten gebracht werden kann.

15. Vorrichtung (10) nach einem der vorhergehenden Ansprüche, **dadurch gekennzeichnet, dass** das zweite Teil aus Polymer besteht.

## Revendications

1. Dispositif (10) destiné à échantillonner un volume faible et précis de liquide, qui comprend un premier élément (12) avec une première ouverture (14) pour l'entrée d'un échantillon de liquide dans une première cavité (16) formant un tunnel capillaire dans le premier élément (12) et avec une deuxième ouverture (18) pour la sortie de l'échantillon de liquide de la première cavité (16), et un deuxième élément (22) avec une deuxième cavité (24) formant un tunnel capillaire destiné à recevoir et à conserver l'échantillon de liquide et ayant une troisième ouverture (28) communiquant avec la deuxième cavité (24), le deuxième élément étant positionné de manière amovible relativement au premier élément (12) de manière à ce que, dans une première position, la deuxième ouverture (18) soit en communication avec la troisième ouverture (28) pour l'entrée de l'échantillon de liquide dans la deuxième cavité, et, dans une deuxième position, la troisième ouverture soit fermée de manière à ce qu'un échantillon de liquide soit piégé dans la deuxième cavité fermée,
**caractérisé en ce que**
le tunnel capillaire comprend un ou plusieurs revêtements de surface hydrophiles.

2. Dispositif (10) selon la revendication 1, **caractérisé en ce que** le revêtement est un réactif.

3. Dispositif (10) selon la revendication 2, **caractérisé en ce que** le réactif est un anticoagulant.

4. Dispositif (10) selon la revendication 3, **caractérisé en ce que** le réactif comprend de l'héparine.

5. Dispositif (10) selon la revendication 3, **caractérisé en ce que** le réactif comprend un sel d'EDTA.

6. Dispositif (10) selon l'une quelconque des revendications précédentes, **caractérisé en ce que** le revêtement est collé à la paroi intérieure du tunnel.

7. Dispositif (10) selon l'une quelconque des revendications précédentes, **caractérisé en ce que** le revêtement est chimiquement lié à la paroi intérieure du tunnel.

8. Dispositif (10) selon l'une quelconque des revendications précédentes, **caractérisé en ce que** le premier élément comprend en outre une troisième cavité (30) destinée à recevoir et contenir au moins une partie du deuxième élément.

9. Dispositif (10) selon l'une quelconque des revendications précédentes, **caractérisé en ce que** le deuxième élément a une forme cylindrique.

10. Dispositif (10) selon la revendication 8, **caractérisé en ce que** le deuxième élément a une forme cylindrique circulaire.

11. Dispositif (10) selon l'une quelconque des revendications précédentes, **caractérisé en ce que** le deuxième élément peut pivoter autour d'un axe de rotation qui est sensiblement perpendiculaire à un axe longitudinal de la deuxième cavité.

12. Dispositif (10) selon l'une quelconque des revendications précédentes, **caractérisé en ce que** le deuxième élément peut être déplacé dans une direction sensiblement perpendiculaire à un axe longitudinal de la deuxième cavité.

13. Dispositif (10) selon l'une quelconque des revendications précédentes, **caractérisé en ce que** l'échantillon de liquide peut être mis en contact avec un autre liquide après le déplacement du deuxième élément.

14. Dispositif (10) selon la revendication 13, **caractérisé en ce que** l'échantillon de liquide peut être mis en contact avec un liquide choisi parmi une pluralité de liquides après le déplacement du deuxième élément dans une position choisie correspondante d'une pluralité de positions correspondantes.

15. Dispositif (10) selon l'une quelconque des revendications précédentes, **caractérisé en ce que** le deuxième élément est formé par un polymère.
